# EUROPEAN PATENT SPECIFICATION

(11) **EP 1 831 115 B1**
(45) Date of publication and mention of the grant of the patent: **17.06.2015**
(21) Application number: 04816682.1
(22) Date of filing: 30.12.2004
(51) Int. Cl.: C12N 1/20, C02F 3/34, C02F 103/30, C12R 1/01

(54) **BIOLOGICAL NEUTRALIZATION OF HIGHLY ALKALINE TEXTILE INDUSTRIAL WASTEWATER**
BIOLOGISCHE NEUTRALISATION VON HOCHALKALISCHEM TEXTILINDUSTRIEABWASSER
NEUTRALISATION BIOLOGIQUE D'EAUX USÉES DE L'INDUSTRIE TEXTILE FORTEMENT ALCALINES

(43) Date of publication of application: 12.09.2007
(73) Proprietor: Council of Scientific and Industrial Research, New Delhi 110 001 (IN)
(72) Inventor: KUMAR, Rita Inst. Genomics & Integrative Biology, Delhi 110 007 (IN); KUMAR, Anil, Inst. Genomics & Integrative Biology, Delhi 110 007 (IN)
(74) Representative: Shah, Punita
(86) International application number: PCT/IN2004/000434
(87) International publication number: WO 2006/070397

(56) References cited:
- US-A1- 2002 064 864
- US-A1- 2004 140 448
- ENZYME AND MICROBIAL TECHNOLOGY, vol. 24, 15 May 1999 (1999-05-15), pages 433-437, XP002347455 USSTONEHAM, MA

## Description

### Field of the invention

The present invention relates to a bacterial isolate, *Kurthia sp.* MTCC 5181 deposited at IMTECH, Sector 39A, Chandigarh, India recognized by Budapest Treaty. The present invention also relates to a process for the neutralization of highly alkaline textile industrial wastewater using the bacterial isolate of the invention.

### Background and prior art

Stringent laws and frequent checks by authorities reflect growing environmental concerns. Thus, for instance, the pH of wastewater of industries such as textiles can deviate only minimally from the neutral point when discharged into a receiving watercourse or sewerage system.

Various chemicals are available to neutralize the high alkaline textile industrial wastewater depending upon the application. In most cases, Sulfuric Acid (H₂S0₄) is used. The end user must consider the concentration to be used, must carefully analyze all the chemistries involved, must review manufacturers' warnings and instructions, and must consider common safety measures for hazardous liquids.

The process of treating wastewaters with chemicals comprises use of either acids or bases or substances capable of forming them on addition to wastewater. Various chemicals are available for industrial neutralization depending upon the application and whether neutralization of an acidic or basic solution is being carried out.

The most commonly used neutralization chemicals for acid or base neutralization are 98% Sulfuric acid and 50% Sodium hydroxide. In many cases these are very good choices. However, there are many considerations when selecting chemicals and these may not always be the best selection. The selection of the chemicals used for the neutralization of an acid or base is almost as important as the design of the neutralization system. Some of the major points to consider in the selection of chemicals are listed below:
- Health and Safety
- Cost and Convenience
- Physical Properties of neutralizing chemicals
- Storage Environment

An explanation of chemical selection criteria is as follows:
Health and Safety: Mixing of chemicals can lead to extreme hazardous/noxious reactions. For example, addition of an acid to cyanide bearing solution results in release of deadly HCN gas.
Cost and Convenience: Acids and bases work in most applications. Sulfuric acid (H₂SO₄), for example, is less costly and more potent than nitric acid. Concentration is also an important consideration in cost assessment. H₂SO₄ for example, can be purchased in concentrations ranging from near 0 to 98%. Higher concentrations are generally less expensive.
Physical Properties: The physical properties of the selected reagent must be considered carefully. 50% Sodium hydroxide (NaOH), for example, begins to freeze at temperatures below 60°F. Decreasing the concentration to 25% eliminates this concern altogether.
Hydrochloric acid (HCl), for example, gasses out severely and is highly corrosive and will attack all metallic objects. Therefore, if HCl is used it must be properly vented or used outdoors where the gasses can easily dissipate.
Storage Environment: Storage issues such as the types of tanks and secondary containment available, familiarity of operators in handling hazardous chemicals, the dangers of refilling storage containers or procedures for transferring from bulk containers are of concern.

The most commonly used neutralizing chemicals are listed below:
- Acids: Sulfuric Acid, Hydrochloric Acid, Nitric Acid, Phosphoric Acid and Carbon Dioxide which forms Carbonic Acid in water
- Bases: Sodium Hydroxide (Caustic Soda), Calcium Hydroxide, Calcium Carbonate (Lime or Limestone), Ammonium Hydroxide

### Neutralization with Acids

- Sulfuric Acid is the most widely used and produced chemical in the world. Available in concentrations ranging from 0% to 98%, sulfuric acid is most economical of all and used universally for neutralization reactions. It is easier and safer to use than HCl or HNO₃ and is more potent than all of the other acids except for phosphoric acid. Sulfuric acid is typically used in concentrations ranging from 25% to 96%. However, 30% to 50% concentrations of sulfuric acid are generally recommended.
- Hydrochloric Acid (HCl), also known as muriatic acid, is the second most commonly used acid in industry, sulfuric acid being the primary choice since it is more effective and relatively inexpensive. At a maximum available concentration of 37%, HCl is about 1/3 as potent as sulfuric acid, thus making it relatively more expensive to use. Depending on temperature and agitation, HCl at concentrations above 10% evolves hydrogen chloride vapors which combine with the water vapors present in the air. The gas thus formed, is highly corrosive and attacks all metallic objects including building structures, sprinkler heads, copper wiring, stainless steel, etc. Therefore, it must be properly vented or used outdoors where the gasses can easily dissipate.
- Nitric Acid (HNO₃) though a widely used chemical in many industries it does not enjoy the popularity of hydrochloric or sulfuric acid, as it is more expensive to use than either of them. Nitric acid evolves noxious gas which on combines with water vapors present in the air. The gas is highly corrosive and attacks all metallic objects including building structures, sprinkler heads, copper wiring, stainless steel, etc. Therefore, it must be properly vented or used outdoors where the gasses can easily dissipate.
- Phosphoric Acid (H₃PO₄), very widely used in the production of agricultural fertilizers and detergent products it is a relatively inexpensive acid. However it still does not compete well with sulfuric and hydrochloric acid as it is a weak acid and does not fully disassociate in water at normal concentrations. This renders it safer to use compared to sulfuric or hydrochloric acid and the evolution of gasses is rare. It tends to buffer neutralization reactions and this makes for a slower reaction that is easier to control. Due to its cost (as compared to sulfuric acid) and availability, phosphoric acid is not commonly used in neutralization system.
- Carbon Dioxide (CO₂), the third most concentrated gas found in earth's atmosphere, CO₂ is itself not an acid. It forms carbonic acid (H₂CO₃) when dissolved in water; and it is this carbonic acid that brings about the neutralization of alkalinity in solution. The most appealing feature of CO₂ is that it will not lower the pH of water below 7.0 for all practical purposes. Additionally CO₂ is non corrosive. However as it is heavier than air asphyxiation is a hazard. Carbon dioxide is difficult to use and its use is limited because the gas must be dissolved into solution to be used. This requires the use of a carbonator, or some method to dissolve the gas into solution Significant out-gassing also occurs, which does not hold a problem unless the process also requires the settling of solids. In cement pouring operations large amounts of alkaline wastewaters are generated. It is an excellent choice for such applications as the site is temporary, the gas is non-hazardous, can be used in-line assuming retention and mixing is considered and is self-buffering so regardless of dosage it will not lower the pH below 7.5-7.0.

### Alkaliphiles

Several microorganisms exhibit more than one pH optimum for growth depending on growth conditions, particularly nutrients, metal ions, and temperature. The term "alkaliphile" is used for microorganisms that grow optimally or very well at pH values above 9. The first paper concerning an alkaline enzyme of alkaliphilic microorganisms was published in 1971 (Horikoshi. K. (1971) Production of alkaline enzymes by alkalophilic microorganisms. Part 1. Alkaline protease produced by Bacillus No, 221. Agric. Biol. Chem. 36, 1407- 1414). Over the past two decades, our studies have focused on the enzymology, physiology, ecology, taxonomy, molecular biology and genetics of alkaliphilic microorganisms. Industrial applications of these microorganisms have also been investigated extensively and some enzymes, such as alkaline proteases, alkaline amylases and alkaline cellulases, have been put to use on an industrial scale (Horikoshi, K. and Akiba, T. (1982) Alkalophilic Microorganisms: A New Microbial World. Springer-Verlag, Heidelberg, Tokyo., Horikoshi, K. (1991) Microorganisms in Alkaline Environments, Kodansha-VCH, Tokyo, Weinheim, New York, Cambridge, Basel).

Subsequently, many microbiologists have published numerous papers on alkaliphilic microorganisms in various fields. Cell surface of alkaliphiles can maintain the intracellular pH values neutral in alkaline environments of pH 10-13. In 1995, using alkaliphilic Bacillus C-125 mutants that are alkaline sensitive developed new host vector systems, and genes responsible for alkaliphily have been investigated (Kudo, T., Hino, M., Kitada, M. and Horikoshi, K. (1990) DNA sequences required for the alkalophily of Bacillus sp. strain C-125 are located close together on its chromosomal DNA. J. Bacteriol. 172, 7282-7283., Seto, Y., Hashimoto, M., Usami, .R., Hamamoto, T., Kudo, T. and Horikoshi, K. (1995) Characterization of a mutation responsible for an alkali-sensitive mutant, 18224, of alkaliphilic Bacillus sp. strain C-125. Biosei. Biotechnol. Biochem. 59, 1364-1366).

Although alkaliphiles have been used for a number of industrial applications, there is no research publication regarding neutralization of textile industrial wastewater using them. US Patent Application No. 09/160422, (1998) discloses a biological neutralization process by using a mixture of bacteria in the presence of sugars (US 2002/0064864).

### Summary of the Invention

The present invention provides a bacterial isolate, *Kurthia sp.* MTCC 5181 deposited at International Depository at IMTECH, Sector 39A, Chandigarh, India recognized by Budapest Treaty. This bacterial strain is capable to bring down the pH of wastewater from 12.0 to 7.0 units within two hours. The neutralization of alkaline textile industrial wastewater by such biotechnological process is highly effective and economical as compared to conventional neutralization by chemical means.

In one embodiment of the invention, the bacterial isolate is capable of growth in a medium of pH range of 10.0-12.0.

In another embodiment of the invention; the bacterial isolate is capable of lowering
the pH (12.0 to 11.5) of textile industrial wastewater to neutral pH (7.5 to 7.0) within a short period (about two hours).

In another embodiment of the invention, the bacterial pellet is used to neutralize high pH (12.0 to 11.5) of textile industrial wastewater to neutral pH (7.5 to 7.0) in ratio ranging from 1:5to 1:10.

In another embodiment of the invention, the bacterial isolate is isolated from soil accumulated in a pipe of an effiuent treatment plant of textile industry located in Chandigarh, India, through which alkaline wastewater has been passed over a period of long time.

In another embodiment of the invention, the bacterial isolate *Kurthia sp.* is Gram - Positive, motile, catalase positive, and capable of reducing nitrate and hydrolyzing starch.

A process of preparing a bacterial isolate of bacterium *Kurthia sp.* MTCC 5181 useful in neutralization of alkaline waste water (pH-12.0 to 11.5) of textile industry and deposited at International Depository at IMTECH, Sector 39A, Chandigarh, India recognized by Budapest Treaty, may comprise:
(a) enriching soil contaminated with bacteria by providing a soil extract in basal carbonate medium for a period of 72-96 hours at 100-120 rpm at 35-37°C in ratio ranges between 1:1-1:2;
(b) culturing the bacteria by using the basal carbonate medium at pH 1.5-12.0 and at 35-37°C for 8-10 hours;
(c) Isolating the said bacteria by centrifuging the culture obtained from step (b) after attaining the heavy growth (O.D. 1.5-2.0) to obtain a pellet of bacterial cell;
(d) dissolving the pellet obtained from step (c) in phosphate buffer;
(e) neutralizing alkaline waste water (pH-12.0 to 11.5) of textile industry by adding the bacterial pellet obtained from step (d) in wastewater.

Contaminated soil may be obtained from a pipe of an effluent treatment plant of textile industry located in Chandigarh, India.

Enrichment of the soil from said site may be done by taking 5-7g of fresh soil in an autoclaved flask containing 100-110ml soil extract, 50µl Candid B (anti-fungal) and basal carbonate medium.

The soil extract may be prepared by centrifuging the sterilized soil mixture at about 4000 rpm for about 20 min.

The sterilized soil mixture may be prepared by dissolving.the dried soil in distilled water in ratio about 1:3 and autoclaved at about 15psi for about one hour.

The basal carbonate medium may contain glucose, bacto-peptone, yeast extract, K₂HPO₄.3H₂O, MgS0₄.7H₂O and sodium carbonate in a ratio of about 1:.5:.5:.13:.02:10 by w/v.

A mixture of soil extract and basal carbonate medium may be used to entrap the maximum bacterial flora of the site.

The ratio between the soil extract and the basal carbonate medium may be in the range of 1:1-1:2.

Isolated bacterial isolates may be cultured under defined conditions such as media, temperature, pH, and carbon source.

All bacterial isolates (total three) may be checked for their neutralizing capability to lower pH of textile wastewater in a short period of time.

Decrease in pH is monitored by pH meter.

A bacterium may be selected which is capable to bring down the pH of alkaline textile wastewater in a short period of about two hours.

The selected bacterium may be cultured under defined conditions by using the basal carbonate medium comprising 1 % (w/v) glucose, 0.5 % (w/v) bacto-peptone, 0.5 % (w/v) yeast extract, 0.13% (w/v) K₂HPO₄.3H₂O, 0.02% (w/v) MgSO₄.7H₂O and 10% (w/v) sodium carbonate followed by incubation at 35-37°C at 120 rpm for 8-10 hours for neutralizing the alkaline textile industrial wastewater.

The grown culture may be centrifuged at 5000-7000 rpm at 1-4°C after attaining the heavy growth O.D. (1.5).

The bacterial pellet may be dissolved in phosphate buffer (pH 6.8).

Neutralization of highly alkaline wastewater of textile industry may be done by adding the bacterial pellet in 200ml wastewater, wherein the lowering of pH from 12.0-11.5 to 7.5 - 7.0 was observed in 2hr to 1.5hr when checked by pH meter.

In another embodiment of the invention, the bacterium, *Kurthia sp.* is used as a whole cell. ,

### Detailed description of the invention

The present invention provides a bacterial isolate *Kurthia sp* MTCC 5181 deposited at International Depository at IMTECH, Sector 39A, Chandigarh, India recognized by Budapest Treaty. The bacterial isolate, *Kurthia sp.* MTCC 5181 is capable of neutralizing the highly alkaline wastewater of textile industry.

The bacterial isolate is capable of growth in a medium of pH range of 10.0 -12.0.

It has been observed that the bacterial isolate is capable of lowering the pH (12.0 to 11.5) of textile industrial wastewater to neutral pH (7.5 to 7.0) within a short period (about two hour). The isolate is used in the form of a bacterial pellet to neutralize high pH (12.0 to 11.5) of textile industrial wastewater to neutral pH (7.5 to 7.0) in ratio ranging from 1:5 to 1:10.

The bacterial isolate is isolated from soil accumulated in a pipe of an effluent treatment plant of textile industry located in Chandigarh; India, through which alkaline wastewater has been passed over a period of long time. The bacterial isolate *Kurthia sp.* is Gram -Positive, motile, catalase positive, and capable of reducing nitrate and hydrolyzing starch.

The process for preparing the bacterial isolate of bacterium *Kurthia sp.* MTCC 5181 comprises:
(a) enriching soil contaminated with bacteria by providing a soil extract in basal carbonate medium for a period of 72-96 hours at 100-120 rpm at 35-37°C in ratio ranges between 1:1-1:2;
(b) culturing the bacteria by using the basal carbonate medium at pH 1.5-12.0 and at 35-37 °C for 8-10 hours;
(c) Isolating the said bacteria by centrifuging the culture obtained from step (b) after attaining the heavy growth *(*O.D.1.5-2.0) to obtain a pellet of bacterial cell;
(d) dissolving the pellet obtained from step (c) in phosphate buffer;
(e) neutralizing alkaline waste water (pH-12.0 to 11.5) of textile industry by adding the bacterial pellet obtained from step (d) in wastewater.

The contaminated soil is obtained from a pipe of an effluent treatment plant of textile industry located in Chandigarh, India. Enrichment of the soil from said site is done by taking 5-7g of fresh soil in an autoclaved flask containing 100-110ml soil extract, 50µl Candid B (anti-fungal) and basal carbonate medium.

The soil extract is prepared by centrifuging the sterilized soil mixture at about 4000 rpm for about 20 min. The sterilized soil mixture is prepared by dissolving the dried soil in distilled water in ratio about 1:3 and autoclaved it at about 15psi for about one hour.

The basal carbonate medium contains glucose, bacto-peptone, yeast extract, K₂HP0₄.3H₂0, MgS0₄.7H₂0 and sodium carbonate in ratio about 1:.5:.5:.13:.02: 10 by w/v. A mixture of soil extract and basal carbonate medium is used to entrap the maximum bacterial flora of the site. The ratio between the said soil extract arid the said basal carbonate medium is between 1:1-1:2.

The isolated bacterial isolates are cultured under defined conditions such as media, temperature, pH, carbon source etc. All the bacterial isolates (total three) are checked for their neutralizing capability to lower the pH of textile wastewater in a short period of time. Decrease in pH is monitored by pH meter. The bacterium is selected based on its capability of lowering the pH of alkaline textile wastewater in a short period of about two hours.

The selected bacterium is cultured under defined conditions by using the bacterial carbonate medium followed by incubation at *37°C at* 120rpm 8 hours for neutralizing the alkaline textile industrial wastewater. The grown culture is centrifuged at 5000-7000rpm at 1-4 °C after attaining the heavy growth O.D. (1.5). The bacterial pellet is dissolved in phosphate buffer (pH 6.8).

Neutralization of highly alkaline wastewater of textile industry is done by adding the bacterial pellet in 200ml wastewater, lowering of pH from 12.0-11.5 to 7.5 - 7.0 was observed in 2hr to 1.5hr as checked by pH meter.

If desired, the bacterium, *Kurthia sp.* is used as a whole cell.

The bacterial strain concerned with the present invention is deposited at International Depository at IMTECH, Sector 39A, Chandigarh, India recognized by Budapest Treaty.

| **S.No.** | **Culture** | **MTCC ID No.** |
|---|---|---|
| *1.* | *Kurthia sp.* | MTCC 5181 |

The above-mentioned bacterial strain exhibits a remarkable capability to neutralize highly alkaline textile industrial wastewater within a short period of 1.5-2 hours under defined conditions. The bacterial strain in the present invention has been isolated from the soil accumulated in the pipe of effluent treatment plant of textile industry located in Chandigarh, India, through which textile, industrial wastewater has been passed over a period of long time. To isolate a potential bacterial isolate, 100-110 ml soil extract; 100-110 ml basal carbonate medium and 50µl Candid B (antifungal). Basal carbonate medium containing 1% (w/v) glucose, 0.5% bacto-peptone, 0.5% yeast extract, 0.13% K₂HPO₄.3H₂O, and 0.02% MgSO₄.7H₂O. Sodium carbonate (10%) was sterilized separately and added to obtain a 1% concentration with initial pH of 10.5 in the medium. The enrichment flask was kept at 100-120 rpm for about 72-96 hours at 35- 37°C.

For the preparation of soil extract, lkg soil was taken and dried at 50°C for 2 hours. 400g of dried soil was dissolved in 960 ml single distilled water and autoclaved at 15 lbs for 1 hour. After autoclaving, the sample was centrifuged at 5000rpm for 10 minutes. The supernatant (extract) was collected and stored in sterile bottle for the preparation of enrichment flask and further use.

The enriched soil sample was serially diluted in 0.85% saline. 10µl from each respective dilution was spread onto agar petri plates containing soil extract and 50% basal carbonate medium. Basal carbonate medium containing 1% (w/v) glucose, 0.5% bacto peptone, 0.5% yeast extract, 0.13% K₂HPO₄.3H₂O, and 0.02% MgSO0₄.7H₂O. Sodium carbonate (10%) was sterilized separately and added to obtain a 1% concentration with initial pH of 10.5 in the medium. The plates thus obtained were incubated at 37 ± 2 °C for 24 - 96 hrs in inverted position.

On the basis of colony morphology and color, total 3 bacterial isolates were selected to check their capability for neutralizing the alkaline wastewater. The single isolated colonies were picked and streaked on fresh plates containing the same medium. The above step was repeated till pure colonies were obtained.

To check the neutralizing capability of the three isolated bacteria, 200 ml textile industrial wastewater of high pH (12.0 -11.5) was taken in glass flask at three places and each bacterial growth was added individually. Decrease in pH was monitored by a pH meter.

Out of three, only one isolate was found capable to grow on high pH (12.0 - 11.5) and bring down the pH of wastewater within a short period of 1.5-2 hours. This bacterium was designated as Kurthia *sp*. and the main characteristic features are:
Bacterium, *Kurthia sp.* is aerobic in nature, is gram positive, shows optimal growth at a temperature in the range of 25-42°C, is capable of growth in a high pH environment (pH 12.0), is capable of hydrolyzing starch, is motile, catalase positive and reduces nitrate.

In a neutralization experiment, textile industrial wastewater was taken from a local textile industry. The bacterium *Kurthia sp.,* as screened above, was inoculated in 200-220 ml basal carbonate medium. The culture was incubated at 35-37°C for 8 hours under shaking conditions (100-120 rpm). After observing the heavy bacterial growth (Optical Density (O.D = 1.5), the culture was centrifuged at 5000-7000 rpm at about 1- 4 °C. The culture pellet was dissolved in about 20ml phosphate buffer according to the size of pellet. This pellet was added in a flask containing 200 ml textile industrial waste water (pH 12.0 - 11.5)). The flask was kept at shaking conditions (100-120rpm). Decrease in pH was observed after 1.5-2 hours. This bacterium, *Kurthia sp.* MTCC 5181 has been capable to bring down the pH from 12-11.5 to 7.5-7 within a short period of 1.5-2 hours. pH was monitored by a pH meter.

The invention further provides a process for the preparation of bacterial growth useful in neutralizing the alkaline wastewater:
a) Enriching the soil of the said site using soil extract and basal carbonate medium to isolate the bacteria having neutralization capability;
b) using the mixture of soil extract and basal carbonate medium containing about 1% (w/v) glucose, about 0.5% bacto-peptone, about 0.5% yeast extract, about 0.13% K₂HPO₄.3H₂O, and 0.02% MgSO₄.7H₂O. Sodium carbonate (about 10%) was sterilized separately and added to obtain a 1% concentration with initial pH of 10.5 to entrap the desired potential bacteria from the said site;
c) culturing the said bacteria isolated from specific site under defined conditions such as media, temperature, pH, carbon source etc.;
d) checking the neutralizing capability of isolated bacterial isolates by inoculating them in 200-220 ml alkaline textile industrial waste water;
e) Decrease in pH was monitored by a pH meter;
f) selecting the bacterial isolate which can neutralize the alkaline waste water in a short period of time;
g) Culturing selected bacterium under defined conditions for neutralizing alkaline textile industrial wastewater. Basal carbonate medium was used to grow a culture. Culture flask was incubated at 35- 37°C for 8 hours at 100-120 rpm in order to obtain heavy growth;
h) centrifuging the resulting culture after attaining the heavy growth O.D. (1.5);
i) collecting the bacterial pellet and dissolving in phosphate buffer;
j) Neutralizing the highly alkaline wastewater of textile industry by adding the bacterial pellet in 200ml wastewater. Lowering of pH from 12.0-11.5 to 7.5-7.0 was observed in 1.5-2 hrs as checked by pH meter.

The following examples are given by way of illustration and therefore should not be construed to limit the scope of the present invention.

### Example 1

In an endeavor of exploring alkaliphilic bacteria, strategic isolation was done to entrap potential bacterial flora from soil accumulated in a pipe of an effluent treatment plant of textile industry located in Chandigarh, India through which alkaline waste water has been passed over a period of long time. Accumulated soil was collected from the said pipe to isolate the bacteria.

To isolate a potential bacterial isolate, 5g soil from the said site was added in the 500 ml autoclaved flask containing 100 ml soil extract; 100 ml basal carbonate medium and 50µl Candid B (antifungal). Basal carbonate medium containing 1% (w/v) glucose, 0.5% bacto peptone, 0.5% yeast extract, 0.13% K₂HPO₄.3H₂O, and 0.02% MgSO₄.7H₂O. Sodium carbonate (10%) was sterilized separately and added to obtain a 1% concentration with initial pH of 10.5in the medium. The enrichment flask was kept at 120rpm for 96 hours at 37°C.

For the preparation of soil extract, lkg soil was taken and dried at *SODC for* 2 hours. 400g of dried soil was dissolved in'960 ml single distilled water and autoclaved at 15 lbs for 1 hour. After autoclaving, the sample was centrifuged at 5000 rpm for 10 minutes. The supernatant (extract) was collected and stored in sterile bottle for the preparation of enrichment flask and further use.

The enriched soil sample was serially diluted in 0.85% saline. 100ul from each respective dilution was spread onto agar petri plates containing soil extract and 50% basal carbonate medium. Basal carbonate medium containing 1% (w/v) glucose, 0.5% bacto peptone, 0.5% yeast extract, 0.13% K₂HPO₄.3H₂O, and 0.02% MgSO₄.7H₂O. Sodium carbonate (10%) was sterilized separately and added to obtain a 1% concentration with initial pH of 10.5 in the medium. The plates thus obtained were incubated at 37 ± 2 °C for 24 - 96 hrs in inverted position.

On the basis of colony morphology and color, total 3 bacterial isolates were selected to check their capability for neutralizing the alkaline wastewater. The single isolated colonies were picked and streaked on fresh plates containing the same medium. The above step was repeated till pure colonies were obtained.

### Example 2

In order to explore the potential bacteria for neutralization of alkaline textile industrial wastewater, total three bacteria were isolated from the pipe through which textile industrial wastewater has been passed over a period of long time. These bacterial isolates were selected to check their capability for neutralizing the alkaline waste water. The single isolated colonies were picked and streaked on fresh agar plates containing the same medium. The above step was repeated till pure bacterial colonies were obtained.

To check the neutralizing capability of the three isolated bacteria, 200 ml textile industrial wastewater of high pH (12.0) was taken in 500ml glass flask at three places and each bacterial growth was added individually. Decrease in pH was monitored by a pH meter (Table 1).

**Table 1. pH reduction of alkaline wastewater by isolated alkaliphilic bacteria.**

| Bacterial Isolates | Reduction in pH of waste water during course of time | | | | | | |
|---|---|---|---|---|---|---|---|
| | 0hr | 0.5hr | 1.0hr | 1.5hr | 2.0hr | 2.5hr | 3.0hr |
| Isolate 1 | 12.12 | 12.11 | 12.11 | 12.10 | 12.10 | 12.01 | 12.00 |
| Isolate 2 | 12,12 | 9.24 | 8.05 | 7.67 | 7.04 | 7.03 | 7.01 |
| Isolate 3 | 12.12 | 12.10 | 12.4 | 11.98 | 11.86 | 11.77 | 11.70 |

### Example 3

On the basis of colony morphology and color, total 3 bacterial isolates were selected to check their capability for neutralizing the alkaline wastewater. The single isolated colonies were picked and streaked on fresh plates containing the same medium. The above step was repeated till pure colonies were obtained.

Out of three, only one isolate (Isolate 2), *Kurthia sp.* was found capable to grow on high pH (12.0) and bring down the pH (7.04) of wastewater within a short period of 2 hours. This bacterium was designated *as Kurthia sp.* and the main characteristic features are:
(a) aerobic in nature
(b) gram positive
(c) shows optimum growth between 25-42°C
(d) capable to grow in a high pH environment (pH 12.0)
(e) capable of hydrolyzing the starch
(f) is motile
(g) is catalase positive
(h) reduces nitrate.

### Example 4

In order to observe the growth of screened bacterium *Kurthia sp.* on a suitable medium, two loops from agar plate of *Kurthia sp.* were streaked onto plates of Nutrient broth medium and Basal carbonate medium. Basal carbonate medium containing 1% (w/v) glucose, 0.5% bacto-peptone, 0.5% yeast extract, 0.13% K₂HPO₄.3H₂O, and 0.02% MgSO₄.7H₂O. Sodium carbonate (10%) was sterilized separately and added to obtain a 1% concentration with initial ph of 10.5 in the medium. The plates thus obtained were incubated at 37 ± 2 °C for 24 - 96 hrs in inverted position.

NB medium containing 2% agar was having original pH about 7 while BCM medium was having original pH 10.5. For increasing the pH of media, Tris-HCL and Na₂CO₃ - NaHCO₃ buffer were used.

**Table 2 shows the growth of Kurthia sp. on NB and BCM medium.**

| **pH values** | **NB medium** | **BCM medium** |
|---|---|---|
| 7.00 | + | + |
| 8.00 | + | ++ |
| 9.00 | ++ | +++ |
| 10.00 | - | ++++ |
| 11.00 | - | ++++ |
| 12.00 | - | +++++ |

| | | |
|---|---|---|
| + Very poor growth ++ Poor growth +++ Good growth ++++ Very good growth | | |

It was observed that BCM medium of high pH is a suitable medium to grow the *Kurthia sp.*

### Example 5

Neutralization of alkaline textile wastewater was also done with lyophilized powder of *Kurthia sp.* Bacterial pellet of 40 ml culture (O.D. = 1.5) was lyophilized and added to 500ml flask containing 200 ml alkaline textile wastewater. Inoculated flask was kept at 37°C for 2 hr. Decrease in pH was observed within two hours (Table 3). The experiment was done in duplicate (Set 1 and Set 2)

**Table 3. pH reduction of alkaline wastewater by lyophilized bacterial powder of Kurthia sp.**

| Bacterial Isolate | Reduction in pH of waste water during course of time | | | | | | |
|---|---|---|---|---|---|---|---|
| | 0hr | 0.5hr | 1.0hr | 1.5hr | 2.0hr | 2.5hr | 3.0hr |
| ***Kurthia** sp.* (Set 1) | 12.11 | 9.56 | 8.22 | 7.45 | 7.23 | 7.02 | 7.01 |
| *Kurthia sp* (Set 2) | 12.11 | 9.33 | 8.35 | 7.67 | 7.12 | 7.03 | 7.00 |

### Advantages

1. The neutralization of alkaline textile wastewater using ***Kurthia sp*** is an economical and effective process. In conventional acid-neutralization process, tones of acid are used for the neutralization while in the developed biological process decrease the cost drastically.
2. The neutralization of alkaline textile wastewater by biological mean is quite safe process as the utilization of acid in large quantities for the neutralization of wastewaters is hot safe for the industry as the strong acid has dangerous effect on the health of workers as well as on the industrial processes. Besides this, use of large quantity of acid also increases the volume of industrial wastewaters to be drained out in the main stream.

## Claims

1. A bacterial isolate, *Kurthia sp.* MTCC 5181 deposited at International Depository at IMTECH, Sector 39 A, Chandigarh, India recognized by Budapest Treaty.

2. A process for neutralizing alkaline waste water of textile industry comprising adding a cell pellet of a bacterial isolate of bacterium *Kurthia sp.* MTCC 5181 thereto to reduce pH of the alkaline waste water from pH12 - 11.5 to neutral pH in the range of 7.5-7.0.

3. The process as claimed in claim 2 wherein the process comprises:
(a) culturing *Kurthia sp.* MTCC 5181 bacteria using basal carbonate medium containing 1 % (w/v) glucose, 0.5 % (w/v) bacto-peptone, 0.5 % (w/v) yeast extract, 0.13% (w/v) K₂HPO₄.3H₂O, 0.02% (w/v) MgSO₄.7H₂O and 10% (w/v) sodium carbonate as a suitable medium to grow the culture;
(b) incubating the culture flask at 35 - 37 °C at 100-120 rpm for 8-10 hours in order to obtain heavy growth;
(c) centrifuging the resulting culture at 5000-7000 rpm at 1-4°C after attaining the heavy growth;
(d) collecting the bacterial pellet;
(e) dissolving the bacterial pellet in phosphate buffer;
(f) neutralizing the highly alkaline waste water of textile industry by adding the whole cell bacterial pellet to 200ml waste water.

4. The process as claimed in claim 3 wherein heavy growth is determined by an optical density of about 1.5 - 2.0.

5. The process as claimed in claim 3 wherein the bacterial pellet is dissolved in phosphate buffer having a pH of about 6.8.

6. The process as claimed in claim 3 wherein the bacterial isolate is sufficient to decrease the pH of waste water from a starting value of pH 12 -11.5 to the end value of pH 7.5 to 7.0.

7. The process as claimed in claim 3 wherein neutralization of highly alkaline waste water is done in 2 - 1.5 hours by a bacterium *Kurthia sp*. MTCC 5181.

8. The process as claimed in claim 3 wherein the bacterium *Kurthia sp*. MTCC 5181 is used as a whole cell.

9. The process as claimed in claim 2 wherein the bacterial pellet is used in a ratio in the range of 1:5 to 1: 10 to the wastewater.

10. The process or isolate as claimed in any one of the preceding claims wherein the bacterium *Kurthia sp.* MTCC 5181 is Gram-Positive, motile, catalase positive, capable of reducing nitrate and hydrolysing starch.

## Patentansprüche

1. Bakterielles Isolat *Kurthia sp.* MTCC 5181, hinterlegt bei der international anerkannten Hinterlegungsstelle International Depository von IMTECH, Sector 39 A, Chandigarh, Indien, anerkannt durch den Budapester Vertrag.

2. Verfahren zum Neutralisieren von alkalischem Abwasser der Textilindustrie, umfassend die Zugabe eines Zellenpellets eines bakteriellen Isolats des Bakteriums *Kurthia sp.* MTCC 5181 dazu, um den pH-Wert des alkalischen Abwassers von 12-11,5 auf einen neutralen pH-Wert im Bereich von 7,5 - 7,0 zu reduzieren.

3. Verfahren nach Anspruch 2, wobei das Verfahren folgendes umfasst:
(a) Züchten von Bakterien *Kurthia sp.* MTCC 5181 unter Verwendung eines basalen Carbonatmediums, das 1 % (w/v) Glucose, 0,5% (w/v) Bacto-Pepton, 0,5% (w/v) Hefeextrakt, 0,13% (w/v) K₂HPO₄.3H₂O, 0,02% (w/v) MgSO₄.7H₂O und 10% (w/v) Natriumcarbonat als geeignetes Medium für die Züchtung der Kultur enthält;
(b) Inkubieren der Kultur-Flasche bei 35 - 37° bei 100 - 120 U./Min. über 8-10 Stunden, um ein starkes Wachstum zu erzielen;
(c) Zentrifugieren der resultierenden Kultur bei 5000 - 7000 U./Min. bei 1 - 4 °C nach Erreichen des starken Wachstums;
(d) Entnahme des bakteriellen Pellets;
(e) Auflösen des bakteriellen Pellets in Phosphatpuffer;
(f) Neutralisieren des hoch alkalischen Abwassers der Textilindustrie durch Zugabe des ganzzelligen bakteriellen Pellets zu 200 ml Abwasser.

4. Verfahren nach Anspruch 3, wobei das starke Wachstum bestimmt wird durch eine optische Dichte von etwa 1,5 - 2,0.

5. Verfahren nach Anspruch 3, wobei das bakterielle Pellet in Phosphatpuffer mit einem pH-Wert von etwa 6,8 aufgelöst wird.

6. Verfahren nach Anspruch 3, wobei das bakterielle Isolat ausreicht, um den pH-Wert von Abwasser von einem Ausgangswert von 12 - 11,5 auf einen Endwert von 7,5 bis 7,0 zu reduzieren.

7. Verfahren nach Anspruch 3, wobei das Neutralisieren des hoch alkalischen Abwassers durch ein Bakterium *Kurthia sp.* MTCC 5181 innerhalb von 2 bis 1,5 Stunden erfolgt.

8. Verfahren nach Anspruch 3, wobei das Bakterium *Kurthia sp.* MTCC 5181 als ganze Zelle verwendet wird.

9. Verfahren nach Anspruch 2, wobei das bakterielle Pellet in einem Verhältnis von 1:5 bis 1:10 zu dem Abwasser verwendet wird.

10. Verfahren oder Isolat nach einem der vorstehenden Ansprüche, wobei das Bakterium *Kurthia sp.* MTCC 5181 grampositiv, motil, katalasepositiv ist, Nitrat reduzieren und Stärke hydrolysieren kann.

## Revendications

1. Isolat bactérien *Kurthia sp.* MTCC 5181, déposé auprès de l'autorité de dépôt internationale de l'IMTECH, secteur 39 A, à Chandigarh, Inde, reconnue par le traité de Budapest.

2. Procédé de neutralisation d'eau usée alcaline de l'industrie textile comprenant l'ajout d'un culot de cellules d'un isolat bactérien de la bactérie *Kurthia sp.* MTCC 5181 à celle-ci pour réduire le pH de l'eau usée alcaline d'un pH dans la plage allant de 12 à 11,5 à un pH neutre dans la plage allant de 7,5 à 7,0.

3. Procédé selon la revendication 2, dans lequel le procédé comprend :
(a) la culture de bactéries *Kurthia sp.* MTCC 5181 en utilisant un milieu carbonate basal contenant 1 % (m/v) de glucose, 0,5 % (m/v) de bacto-peptone, 0,5 % (m/v) d'extrait de levure, 0,13 % (m/v) de K₂HPO₄·3 H₂O, 0,02 % (m/v) de MgSO₄·7 H₂O et 10 % (m/v) de carbonate de sodium en tant que milieu approprié pour développer la culture ;
(b) l'incubation de la fiole de culture à 35 à 37 °C à 100 à 120 tours/minute pendant 8 à 10 heures afin d'obtenir une croissance importante ;
(c) la centrifugation de la culture résultante à 5 000 à 7 000 tours/minute à 1 à 4 °C après l'obtention de la croissance importante ;
(d) le recueillement du culot bactérien ;
(e) la dissolution du culot bactérien dans un tampon phosphate ;
(f) la neutralisation de l'eau usée hautement alcaline de l'industrie textile par ajout du culot bactérien de cellules entières à 200 ml d'eau usée.

4. Procédé selon la revendication 3, dans lequel la croissance importante est déterminée par une densité optique d'environ 1,5 à 2,0.

5. Procédé selon la revendication 3, dans lequel le culot bactérien est dissous dans un tampon phosphate ayant un pH d'environ 6,8.

6. Procédé selon la revendication 3, dans lequel l'isolat bactérien est suffisant pour diminuer le pH de l'eau usée d'une valeur de départ de 12 à 11,5 à la valeur finale de 7,5 à 7,0.

7. Procédé selon la revendication 3, dans lequel la neutralisation de l'eau usée hautement alcaline est effectuée en 2 à 1,5 heures par une bactérie *Kurthia sp.* MTCC 5181.

8. Procédé selon la revendication 3, dans lequel la bactérie *Kurthia sp.* MTCC 5181 est utilisée sous la forme d'une cellule entière.

9. Procédé selon la revendication 2, dans lequel le culot bactérien est utilisé en un taux dans la plage allant de 1:5 à 1:10 par rapport à l'eau usée.

10. Procédé ou isolat selon l'une quelconque des revendications précédentes, dans lequel la bactérie *Kurthia sp.* MTCC 5181 est à Gram positif, motile, catalase positive, capable de réduire les nitrates et d'hydrolyser l'amidon.
